# EUROPEAN PATENT APPLICATION

(11) **EP 4 458 373 A1**
(43) Date of publication of application: **06.11.2024**
(21) Application number: 23275075.2
(22) Date of filing: 03.05.2023
(51) Int. Cl.: A61K 39/09, A61K 9/127, A61K 39/00

(54) **PROCESS FOR OBTAINING LIPOSOMAL PARTICLES COMPRISING AT LEAST ONE PNEUMOCOCCAL SURFACE PROTEIN A (PSPA) AND AN ADJUVANT MOLECULE FORMULATED IN NANOCOMPOSITE MICROPARTICLE CARRIERS (NCMP), LIPOSOMAL PARTICLES OBTAINED BY SAID PROCESS, USE THEREOF AND IMMUNOGENIC COMPOSITION**

(71) Applicant: INSTITUTO BUTANTAN, 05503-000 São Paulo (BR); Liverpool John Moores University, Liverpool Merseyside L3 5AH (GB)
(72) Inventor: DA COSTA RODRIGUES, Tasson, 06767-030 TABOÃO DA SERRA (BR); MAIMONI GONÇALVES, Viviane, 05416-010 Sao Paulo (BR); NAMIE MIYAJI, Eliane, 05641-000 Sao Paulo (BR); BORGES FIGUEIREDO, Douglas, 04516-001 Sao Paulo (BR); SALEEM, Imran, Liverpool, L3 3AF (GB); KANEKO, Kan, Liverpool, L3 3AF (GB)
(74) Representative: Murgitroyd & Company

(57) **Abstract**

The present invention relates to a process for obtaining liposomal particles comprising at least one pneumococcal surface protein A (PspA) and an adjuvant molecule formulated in nanocomposite microparticle carriers (NCMP), wherein the process comprises the steps of (a) preparing liposomal particles (LPs) having from 50 to 90% in moles of dipalmitoylphosphatidylcholine (DPPC); and from 10 to 50% in moles of 3β-[N-(N',N'-dimethylaminoethane)-carbamoyl]cholesterol (DC-Col); and from 0.00100 to 0.00775% in moles of αGalCer to organic phase; and contains a concentration of 0.3 to 2.0 mg/mL of a solution of one or more PspA; (b) centrifuging the LPs obtained in step "a" to remove non-encapsulated protein; (c) resuspending said LPs in a sugar solution; and (d) drying the solution obtained in step "c" by atomization spray-drying (SD) to obtain at the end a powder containing NCMPs carrying the LPs. In addition, the present invention relates to the liposomal particles (LP/NCMPs) obtained from said process the use thereof and the immunogenic composition comprising said LP/NCMPs.

## Description

### Application Field:

The present invention is inserted in the field of preparations for medical purposes, more specifically, in the field of vaccines, since it refers to a liposomal powder formulation as a mucosal vaccine against infections caused by *pneumococcus.*

### Background:

*Streptococcus pneumoniae,* or pneumococcus, is a transient constituent of the human microbiota, but in some cases, it can cause disease. Vaccination is the most important way to control pneumococcal infections and the currently licensed vaccines are based on immunization with the capsule polysaccharide (PS). After the introduction of the conjugate vaccines (PCV) that contain PS conjugated to a carrier protein, a reduction in invasive pneumococcal disease (IPD) and protection by herd effect were observed.

However, infections caused by *pneumococci* continue to be a huge burden in public health, despite the availability of polysaccharide conjugate vaccines (PCVs). There are more than 100 pneumococcal serotypes, each one with a different capsular PS, and the commercially available vaccines cover up to 20 serotypes.

As previously mentioned, after the introduction of PCVs, there was an impressive reduction in invasive pneumococcal disease (meningitis, bacteremia, and invasive pneumonia) caused by serotypes included in the vaccine formulations. On the other hand, there was substitution by disease caused by non-vaccine serotypes. Furthermore, licensed vaccines are not very effective against non-invasive disease, such as pneumonia, otitis and sinusitis. Another drawback of PCVs is the high production cost due to the high number of serotypes included in the formulation and the low efficiency of the conjugation process.

For these reasons, a new generation of serotype-independent vaccines has gained importance, and *"Pneumococcal surface protein A"* (PspA) is among the most important protein vaccine candidates. PspA is expressed in all clinical isolates and shows some diversity, being divided into family 1 (clades 1 and 2), family 2 (clades 3, 4 and 5), and family 3 (clade 6). Since PspA from families 1 and 2 are the most prevalent, a broad coverage vaccine must induce protection against both families.

Thus, in view of the aforementioned technical problem, the present invention proposes a formulation of liposome particles containing an adjuvant molecule (α-galactosyl ceramide - αGalCer) and two variants of the antigen PspA, wherein the antigens were produced as recombinant proteins. The formulation was spray-dried, with the production of a vaccine for mucosal delivery that is stable at room temperature, with low production cost and broad coverage of pneumococcal strains.

Therefore, the formulations of the present invention have the potential to be used as a mucosal vaccine with low cost and broad coverage against pneumococcal infections.

Some prior art documents describe the development of formulations against pneumococcal infections comprising certain fragments of PspA and αGalCer as adjuvant.

International patent application No. PCT/GB20119/053218, published under N° WO 2020/099869 (A1) on May 22, 2020, in the name of LIVERPOOL JOHN MOORES UNIVERSITY, entitled: *"NANOPARTICLES AND USES THEREOF"* refers to a nanoparticle comprising a polymer, α-galactosylceramide (α-GalCer) and chitosan, and additionally one or more PspA antigens. Differently, the present invention proposes a formulation of liposomal nanoparticles, comprising two variants of the PspA antigen, and, consequently, presenting a process for obtaining nanoparticles different from that disclosed in this international patent application.

North-American patent application No. US 2008/317769 (A1), published on December 25, 2008, in the name of SEOUL NAT UNIV R&DB FOUND, entitled: *"VACCINE COMPOSITION COMPRISING ALPHA-GALACTOSYLCERAMIDE AS AN ADJUVANT FOR INTRANASAL ADMINISTRATION"* describes a vaccine composition comprising an antigen and an effective dose of alpha-galactosylceramide (alphaGalCer) as an adjuvant for intranasal administration. Differently, the present invention proposes a formulation of liposomal nanoparticles, comprising two variants of the PspA antigen, and, consequently, presenting a process for obtaining nanoparticles different from that disclosed in this North-American patent application.

North-American patent application No. US 2009/214596 (A1), published on August 27, 2009, in the name of RIKEN, entitled: *"NASAL VACCINE"* refers to a nasal vaccine capable of intranasally inducing an increase in antigen-specific immunoglobulin seen in infection by viruses and the like, and particularly to a nasal vaccine containing the compound with NKT cell activating action as an active ingredient. However, the present invention distances itself from the aforementioned North-American document by describing a formulation of liposomal nanoparticles, comprising two variants of the PspA antigen, and, consequently, presenting a process for obtaining nanoparticles different from that disclosed in the prior art, with αGalCer being used in the formulation as an adjuvant.

The article on behalf of Deng S, et al., entitled: "A PEPTIDE-FREE, LIPOSOME-BASED OLIGOSACCHARIDE VACCINE, ADJUVANTED WITH A NATURAL KILLER T CELL ANTIGEN, GENERATES ROBUST ANTIBODY RESPONSES IN VIVO", published in Chem Sci; 5(4):1437-1441, on April 2014, under the DOI: https://doi.org/10.1039/C3SC53471E, refers to a simple liposome-based approach to generate multivalent carbohydrate vaccines, and instead of an antigenic peptide or protein, a potent natural killer T-cell antigen was used. Differently, the present invention proposes a formulation of powdered liposomal nanoparticles, comprising two variants of the PspA antigen, and, consequently, presenting a process for obtaining nanoparticles different from that disclosed in this article.

The article on behalf of Rui Tada, et al., entitled: "NASAL VACCINATION WITH PNEUMOCOCCAL SURFACE PROTEIN A IN COMBINATION WITH CATIONIC LIPOSOMES CONSISTING OF DOTAP AND DC-CHOL CONFERS ANTIGEN-MEDIATED PROTECTIVE IMMUNITY AGAINST STREPTOCOCCUS PNEUMONIAE INFECTIONS IN MICE", published in International Immunopharmacology, Volume 61, 2018, Pages 385-393, ISSN 1567-5769, under the DOI: https://doi.org/10.1016/j.intimp.2018.06.027, refers to a nasal pneumococcal vaccine composed of pneumococcal surface protein A (PspA) and cationic liposomes composed of 1,2-dioleoyl-3-trimethylammonium-propane (DOTAP) and cholesteryl 3β-N- (dimethylaminoethyl) carbamate (DC-chol) - (DOTAP/DC-chol liposome). Differently, the present invention proposes a formulation comprising αGalCer as an adjuvant and two variants of the PspA antigen, and, consequently, present a process for obtaining nanoparticles different from that disclosed in this article.

Thus, although formulations against pneumococcal infections comprising certain fragments of PspA and αGalCer as adjuvant already exist, there is no motivation in the state of the art to obtain a formulation comprising liposomal particles composed of Dipalmitoylphosphatidylcholine (DPPC), 3β-[N-(N ' ,N '-dimethylaminoethane)-carbamoyl]cholesterol (DC-Chol) and alpha-galactosylceramide (αGalCer) containing the protein antigens PspA1 (family 1) and PspA4Pro (family 2), and the process of obtain thereof, as proposed by the present invention.

Therefore, based on the cited state of the art, the present invention can be considered a new option among others already known. The strengths of the formulation of the present invention would be pulmonary immunization with a dry formulation stable at room temperature, the possibility of scaling up the liposome production process by microfluidic technique, increasing the level of specific antibodies and broader protection against pneumococcal isolates through the inclusion of the adjuvant alpha-galactosyl ceramide.

### Summary of the invention:

As already mentioned, the present invention will provide significant advantages for broad-spectrum protection against different pneumococcal isolates.

In a first aspect, the present invention relates to a process for obtaining liposomal particles comprising at least one pneumococcal surface protein A (PspA) and an adjuvant molecule formulated in nanocomposite microparticle carriers (NCMP), wherein the process comprises the steps of (a) preparing liposomal particles (LPs) having from 50 to 90% in moles of dipalmitoylphosphatidylcholine (DPPC), preferably 69.99900%; and from 10 to 50% in moles of 3β-[N-(N',N'-dimethylaminoethane)-carbamoyl]cholesterol (DC-Chol), preferably 30.00000%; and from 0.00100 to 0.00775% in moles of αGalCer, preferably 0.00100%, to organic phase; and contains a concentration of 0.3 to 2.0 mg/mL of a solution of one or more PspA, preferably 0.3 mg/mL; (b) Centrifuging the LPs obtained in step "a" to remove non-encapsulated protein; (c) resuspending said LPs in a sugar solution; and (d) drying the solution obtained in step "c" by atomization spray-drying (SD) to obtain at the end a powder containing NCMPs carrying the LPs.

In a second aspect, the present invention relates to liposomal particles (LP/NCMPs) obtained from the process of the present invention, wherein said LP/NCMPs comprise from 50 to 90% in moles of dipalmitoylphosphatidylcholine (DPPC), and from 10 to 50% in moles of 3β-[N-(N',N'-dimethylaminoethane)-carbamoyl]cholesterol (DC-Chol), and from 0.00100 to 0.00775% in moles of an adjuvant molecule consisting of αGalCer forming the organic phase; from 0.3 to 2.0 mg of one or more PspA; and a sugar.

In a third aspect, the present invention relates to the use of LP/NCMPs of the present invention, wherein it is for the preparation of an immunogenic composition for comprehensive protection against pneumococcus expressing PspA from family 1 and family 2, wherein the immunogenic composition is preferably a vaccine powder for mucosal administration targeting the lungs.

In a fourth aspect, the present invention relates to an immunogenic composition that comprises the LP/NCMPs of the present invention, and a pharmaceutically acceptable carrier and/or adjuvant.

### Brief description of the figures:

The present invention, along with its further advantages, may be better understood by referring to the attached images and the following description.
Figure 1 illustrates the production of liposomes using NanoAssemblr^{®}, wherein A refers to the NanoAssemblr^{®}, and B refers to the process for producing liposomes.
Figure 2 refers to morphological characterization of LP/NCMPs containing PspA1 and PspA4Pro, wherein mixtures of DPPC:DC-Chol (A) and DPPC:DC-Chol:αGalCer (B) were used to produce empty particles (1A and 1B), containing PspA1 (2A and 2B), PspA4Pro (3A and 3B) and the combination PspA1+PspA4Pro (4A and 4B).
Figure 3 graphically illustrates the evaluation of antibody binding to PspA1 and PspA4Pro released from LP/NCMPs, wherein (A) refers to PspA1 and particles of DPPC:DC-Chol, (B) refers to PspA4Pro and particles of DPPC:DC-Chol, (C) refers to PspA1 and particles of DPPC:DC-Chol:αGalCer, and (D) refers to PspA4Pro and particles of DPPC:DC-Chol:aGalCer, and the combination of PspA1+PspA4Pro refers to (9A, 9B, 9C and 9D).
Figure 4 graphically illustrates the evaluation of lactoferrin-binding activity by proteins released from LP/NCMPs, wherein (A) refers to particles of DPPC:DC-Chol, (B) refers to particles of DPPC:DC-Chol:αGalCer, PspA1 refers to 6A and 5B, PspA4Pro refers to 5A and 6B and the combination of PspA1+PspA4Pro refers to 7A and 7B.
Figure 5 graphically illustrates the induction of serum antibodies by different LPs composed of DPPC:DC-Chol or DPPC:DC-Chol:aGalCer and formulated as LP/NCMPs containing PspA4Pro for determination of anti-PspA4Pro IgG antibody titer after 14 days of the first (A) and second (B) dose.
Figure 6 graphically illustrates the induction of specific serum antibodies after immunization with LPs composed of DPPC:DC-Chol:aGalCer containing PspA1 and PspA4Pro and formulated as LP/NCMPs, wherein (A) refers to anti-PspA1 IgG antibodies, and (B) refers to anti-PspA4Pro IgG antibodies.
Figure 7 graphically illustrates the induction of specific mucosal (vaginal wash) IgG and IgA antibodies after immunization with LPs composed of DPPC:DC-Chol:αGalCer containing PspA1 and PspA4Pro and formulated as LP/NCMPs, wherein (A) refers to the anti-PspA1 IgA, (B) refers to the anti-PspA4Pro IgA, (C) refers to anti-PspA1 IgG, and (D) refers to anti-PspA4Pro IgG.
Figure 8 graphically illustrates the ratio between PspA4Pro-specific serum IgG1 and IgG2a after immunization.
Figure 9 graphically illustrates the surface binding to different pneumococcal strains of serum IgG antibodies induced by PspA-containing LPs composed of DPPC:DC-Chol:aGalCer and formulated as LP/NCMPs, wherein (A) refers to pneumococcal strain EF3030 (serotype 19F, PspA1), (B) A66.1 (serotype 3, PspA2), (C) M10 (serotype 11A, PspA3), (D) 3JYP2670 (serotype 3, PspA4) and (E) ATCC6303 (serotype 3, PspA5).
Figure 10 graphically illustrates the survival time curve of mice immunized with LPs composed of DPPC:DC-Chol:aGalCer and formulated as LP/NCMPs containing PspA1 and PspA4Pro after challenge with the ATCC6303 strain (serotype 3, PspA5, family 2).
Figure 11 graphically illustrates the survival time curve of mice immunized with LPs composed of DPPC:DC-Chol:aGalCer and formulated as LP/NCMPs containing PspA1 and PspA4Pro after challenge with the A66.1 strain (serotype 3, PspA2, family 1).
Figure 12 graphically illustrates the percentage of memory resident CD4+ T cells in the lungs after challenge.

### Detailed description of the invention:

While the present invention may be susceptible to different embodiments, a preferred embodiment is shown in the following detailed discussion, with the understanding that the present embodiment is to be considered as an exemplification of the principles of the invention and is not intended to limit the present invention to what has been described in this specification.

The present invention relates to a process for obtaining liposomal particles comprising at least one pneumococcal surface protein A (PspA) and an adjuvant molecule formulated in nanocomposite microparticle carriers (NCMP), which comprises the following steps:
a) Preparing liposomal particles (LPs) from the following substeps:
   a.1) Mixing from 50 to 90% in moles of dipalmitoylphosphatidylcholine (DPPC), preferably 69.99900%; and from 10 to 50% in moles of 3β-[N-(N',N'-dimethylaminoethane)-carbamoyl]cholesterol (DC-Col), preferably 30.00000%, in 1 mL ethanol to organic phase;
   a.2) Adding from 0.00100 to 0.00775% in moles of an adjuvant molecule consisting of αGalCer, preferably 0.00100%, in said mixture obtained in step "a";
   a.3) Adding 10 to 20 mg/mL of said mixture obtained in step "b", preferably 15 mg/mL, in 1 mL of organic phase to 5 mL of aqueous solution, which contains a concentration of 0.3 to 2.0 mg/mL of a solution of one or more PspA, preferably 0.3 mg/mL;
b) Centrifuging the LPs obtained in step "a" to remove non-encapsulated protein;
c) Resuspending said LPs in a sugar solution;
d) Drying the solution obtained in step "c" by atomization spray-drying (SD) to obtain at the end a powder containing NCMPs carrying the LPs.

In one embodiment of the invention, said step "a" is performed by microfluidic-based preparation method. In a preferred embodiment, microfluidic-based method is used with the NanoAssemblr^{®} benchtop device (Precision Nanosystems). Syringes containing 1 mL of the lipids in ethanol or 5 mL of the proteins in aqueous solution were sealed, heated at 50°C for 5 minutes and coupled to the equipment. Total flow rate was 9.5 mL/min and flow rate ratio was 10.5:1 (aqueous:organic).

In step "a", PspA is selected from the group consisting of PspA1 (GenBank AY082387), PspA4Pro (GenBank EF649969.1), or a mixture thereof.

Preferably, PspA1 consists of the amino acid sequence as set out in SEQ ID NO:1, which is translated by the PspA1 gene sequence as set out in SEQ ID NO:2.

Preferably, the PspA4Pro consists of the amino acid sequence as set forth in SEQ ID NO:3, which is translated by the PspA4Pro gene sequence as set out in SEQ ID NO:4.

The amino acid sequences of PspA1 and PspA4Pro have some differences, mainly in the final region of the N-terminal portion, where the clade defining region is located, and in the initial region of the N-terminal portion, with PspA of clade 4 presenting a longer amino acid sequence than clade 1 PspA. The N-terminal region of this protein is charged, being responsible for interacting with the capsule and preventing the deposition of C3b from the complement system. The difference between the two clades both in the amino acid sequence and in the charge of these residues can lead to different interactions with lipid compounds. Both the DPPC and DC-Chol molecules have a longer nonpolar part and a shorter hydrophilic part. This molecule composition is essential for the interaction between them to form bilayers discs and followed by the formation of spherical liposomes.

During the mixing process, on a nanoliter scale, the sudden change in the charge of the medium and the slow decrease in the organic solvent content are responsible for triggering the self-assembly of the liposomes.

In one embodiment of the invention, the PspA solution comprises from 0.3 to 2.0 mg/mL of PspA1, preferably 0.3 mg/ml; or from 0.3 to 2.0 mg/mL of PspA4Pro, preferably 0.3 mg/ml; or from 0.3 to 2.0 mg/mL of 1:1 (PspA1:PspA4Pro), preferably 0.3 mg/mL.

Still in step "a", the aqueous solution is composed of deionized water or a solution of lyophilized PspA1, or PspA4Pro or a mixture of PspA1 and PspA4Pro resuspended in water, dialyzed in PBS and diluted in deionized water at a concentration of 0.3 mg/mL.

In step "b" centrifugation is, preferably, at 84,035 g for 35 minutes at a temperature from 18 to 25 °C.

In step "c", the sugar is selected from the group consisting of trehalose, mannitol, sucrose or lactose.

Preferably, in step "c" the sugar is used in a ratio of 20:1 (sugar:lipid).

In one embodiment of the invention, trehalose is preferably used as sugar. Trehalose is a sugar with possible application in the formulation of nanoparticle microcarriers to favor the redispersion of the particles. Furthermore, this excipient favored the redispersion of the particles, in addition to protecting the liposomes with the molecule of interest and maintaining a low percentage of moisture in the formulation.

Thus, with the preferential use of trehalose as a drying excipient for LP/NCMPs containing PspA1 and/or PspA4Pro in step "c", an improvement in the average particle size was observed.

After step "d" of SD, the particle size and polydispersity index (PdI) values remained stable, despite some variation. The yield of the drying process, for the formulation obtained, was between 60 and 70%, resulting in particles with a final moisture content between 4 and 5%.

Finally, the morphology of the LP/NCMPs formed by SD and analyzed by Scanning Electron Microscopy (SEM) was quite homogeneous, presenting spheric particles of sizes that vary in the micrometer scale, favoring the pulmonary deposition of the obtained formulations. In addition, the drying process was able to preserve the characteristics of the LPs, since the values obtained for size and PdI after resuspension of LP/NCMPs in water are similar to those observed shortly after the production of LPs using the microfluidic technique.

It is worth mentioning that the formulation of LPs in NCMPs by SD is essential to increase the stability of the formulation, since there is a significant reduction of free water during the drying process. Furthermore, drying the LPs into NCMPs favors the deposition of these particles in the lung due to the aerodynamic properties of NCMPs, since very small particles can be easily exhaled during breathing.

Therefore, particles with relatively similar sizes were obtained, average yield around 60% and maintenance of a residual moisture below 5%.

In addition, the present invention relates to said liposomal particles (LP) comprising at least one pneumococcal surface protein A (PspA) and an adjuvant molecule formulated in nanocomposite microparticle carriers (NCMP).

Thus, said LP/NCMPs comprise:
- from 50 to 90% in moles of dipalmitoylphosphatidylcholine (DPPC), and from 10 to 50% in moles of 3β-[N-(N',N'-dimethylaminoethane)-carbamoyl]cholesterol (DC-Chol), and from 0.00100 to 0 .00775% in moles of an adjuvant molecule consisting of αGalCer forming the organic phase;
- from 0.3 to 2.0 mg of one or more PspA; and
- sugar.

In one embodiment of the invention, the LP/NCMPs comprise 69.99900% moles of DPPC, 30.00000% moles of DC-Chol, and 0.00100% moles of αGalCer forming the organic phase.

In one embodiment of the invention, the LP/NCMPs comprises a solution of 0.3 mg/mL of PspA1; or 0.3 mg/mL of PspA4Pro; or 0.3 mg/mL of 1:1 of (PspA1:PspA4Pro).

PspA is selected from the group consisting of PspA1 (GenBank AY082387), PspA4Pro (GenBank EF649969.1), or a mixture thereof.

Preferably, PspA1 consists of the amino acid sequence as set out in SEQ ID NO:1, which is translated by the PspA1 gene sequence as set out in SEQ ID NO:2.

Preferably, the PspA4Pro consists of the amino acid sequence as set forth in SEQ ID NO:3, which is translated by the PspA4Pro gene sequence as set out in SEQ ID NO:4.

In one embodiment of the invention, the LP/NCMPs comprises a sugar in the ratio of 20:1 (sugar:lipid).

The sugar is selected from the group consisting of trehalose, mannitol, sucrose or lactose. In one embodiment of the invention, trehalose is preferably used as the sugar.

In a preferred embodiment of the invention, the LP/NCMPs are in powder format, dried by atomization spray-drying (SD).

The average size of said LP/NCMPs ranges from 172 to 366 nm, preferably 172 nm.

The PdI of said LP/NCMPs ranges from 0.155 to 0.308, preferably 0.200.

The zeta potential of said LP/NCMPs ranges from +16.93 to +39.95, preferably +38.00.

The encapsulation efficiency of said LP/NCMPs ranges from 75.04 to 99.99%, preferably 90.00%.

Additionally, said LP/NCMPs showed induction of high titers of serum IgG antibodies against PspA family 1 and family 2. In addition, protection was demonstrated against challenge with pneumococci expressing the 2 families of PspA most commonly expressed in isolates clinical.

Thus, in a preferred embodiment of the invention, the aforementioned LP/NCMPs are for use as a vaccine with comprehensive protection against pneumococcus.

In addition, the present invention relates to the use of said LP/NCMPs for the preparation of an immunogenic composition for comprehensive protection against pneumococcus expressing PspA from family 1 and family 2 .

Preferably the immunogenic composition is a vaccine powder for mucosal administration.

Additionally, the present invention relates to an immunogenic composition comprising said LP/NCMPs and a pharmaceutically acceptable carrier and/or adjuvant.

In one embodiment of the invention, the immunogenic composition is a vaccine powder.

In one embodiment of the invention, said immunogenic composition is for use as a vaccine with comprehensive protection against pneumococcus expressing PspA from family 1 and family 2 .

A pharmaceutically acceptable carrier means a non-toxic, inert solid, semi-solid liquid excipient, diluent, formulation adjuvant of any type, or simply a sterile aqueous medium such as saline. Some examples of materials that can serve as pharmaceutically acceptable carriers and/or adjuvants are sugars, such as lactose, glucose and sucrose, starches, such as corn starch and potato starch, cellulose and its derivatives, such as sodium carboxymethylcellulose, ethylcellulose and cellulose acetate, cyclodextrin; oils, such as peanut oil, cottonseed oil, sunflower oil, sesame oil, olive oil, corn oil and soybean oil; glycols such as propylene glycol, polyoils such as glycerin glycol, sorbitol, mannitol and polyethylene; esters, such as ethyl laurate, ethyl oleate, agar; buffering agents such as aluminum hydroxide and magnesium hydroxide; alginic acid; pyrogen-free water; isotonic saline, Ringer's solution; Ethyl alcohol and phosphate buffer solutions, as well as other compatible non-toxic substances used in pharmaceutical formulations.

Thus, a pharmaceutically acceptable carrier and/or adjuvant is any pharmaceutically acceptable carrier or adjuvant known from the state of the art for obtaining a vaccine.

In one embodiment, said immunogenic composition is administered via the mucosal route, targeting the lungs.

Therefore, in order to elucidate the present invention, experimental results and embodiments are presented below in order to demonstrate the inventiveness of using the LP/NCMPs of the present invention.

### EXAMPLES

### - Preparation of liposomes:

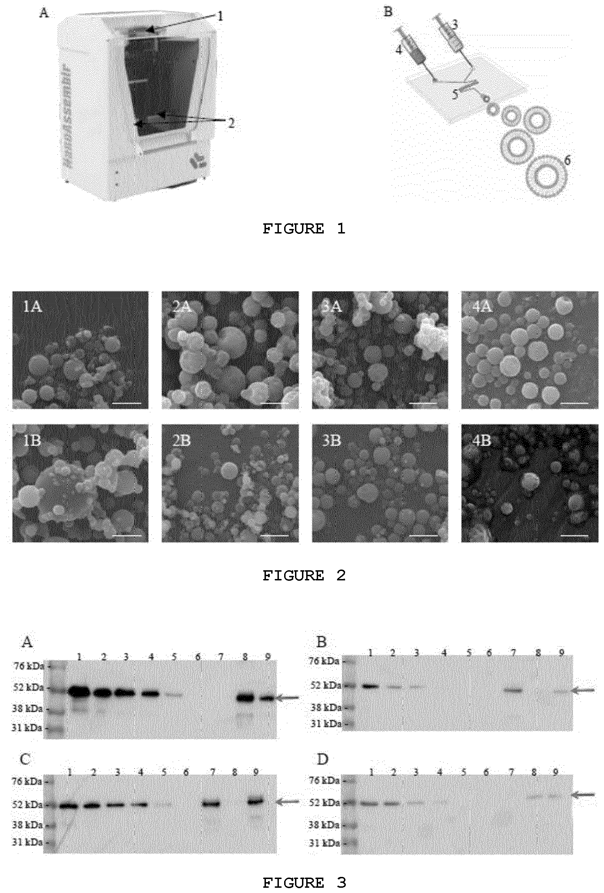

LPs were prepared using microfluidic-based preparation method with the NanoAssemblr^{®} benchtop device *(Precision Nanosystems)* (Figure 1). For optimization assays, LPs containing *Bovine serum albumin* (BSA) were produced. After determining the parameters, a final formulation of each LP was produced, containing a mixture of the two purified proteins (LP PspA1+PspA4Pro) or each protein separately (LP PspA1 and LP PspA4Pro), in addition to LPs without inclusion of proteins. The DPPC:DC-Chol (LP A) and DPPC:DCChol:αGalCer (LP B) lipid mixture at complementary percentages was dispersed in analytical grade ethanol (Merck). Another solution was prepared by diluting the proteins (BSA, PspA1, PspA4Pro or PspA1+PspA4Pro mixture) in deionized water following the final concentration of the formulation. A 1 mL syringe was filled with the lipid mixture and a 5 mL syringe was filled with the protein solution. Each of the syringes was sealed, warmed in water at 50°C for 5 minutes and attached to the entry position of the microfluidic cartridge (*Precision Nanosystems*), previously installed in the device (Figure 1).

Using specific software to control the machine (*Precision Nanosystems*), the total flow rate (TFR) and the flow rate ratio (FRR) were adjusted and kept them constant.

For the formulations of LP A and LP B, the parameters were 9.5 mL/min for TFR and 10.5:1 (aqueous phase:organic phase) for FRR. The formulations were recovered and transferred to specific tubes resistant to ultracentrifugation. The samples were stored at room temperature until they were separated in an ultracentrifuge (Beckman Coulter) at a rotation speed of 84,035 g for 35 minutes. The supernatants of the formulations were transferred to clean tubes and the precipitates were resuspended in distilled water or SD solution. Samples were stored at room temperature until characterization.

After preparing the formulations, the particles were characterized and the results obtained (average size, PdI, zeta potential, encapsulation efficiency) were used to feed the mathematical model into the software to perform factorial regression analysis, using the values of each answer and correlating these values to the factors defined by the Design of experiment (DoE) - (lipid concentration (ConL), composition of liposomes (ComL) and protein concentration (ConP)).

Thus, a new DoE was performed to determine the ideal formulation of DPPC:DC-Chol:aGalCer (formulation LP B), maintaining the machine parameters for TFR and FRR, the concentration of 15 mg total lipids in the organic phase, and using the concentration of protein of 0.3 mg/mL BSA in the aqueous phase.

In Table 1 are the compositions of the LPs and the responses observed in this DoE. With these answers and using the formulation optimization tool in the Minitab 19 software (Minitab, LLC), the formulation was determined by following the order of importance, of greater encapsulation efficiency, and smaller average size, PdI and particle charge.

**Table 1 - Responses produced in different compositions of LPs containing DPPC:DC-Chol:aGalCer.**

| Combination | Composition of liposomes | | | Size (nm) | PdI | Charge (mV) | EE (%) |
|---|---|---|---|---|---|---|---|
| | DPPC (% moles) | DC - Chol (% moles) | αGalCer (% moles) | | | | |
| N1 | 70.00000 | 29.9990 | 0.00100 | 140.08 | 0.255 | +21.60 | 35.99 |
| N2 | 69.99900 | 29.9910 | 0.00100 | 222.24 | 0.376 | +26.93 | 33.47 |
| N3 | 70.00000 | 29.9900 | 0.00100 | 373.44 | 0.574 | +34.98 | 30.78 |
| N4 | 69.99900 | 30.0000 | 0.00100 | 458.34 | 0.512 | +32.00 | 34.08 |
| N5 | 69.99950 | 29.9950 | 0.00550 | 66.34 | 0.455 | +26.89 | 34.95 |
| N6 | 69.99975 | 29.9970 | 0.00325 | 564.31 | 0.583 | +38.03 | 27.79 |
| N7 | 69.99925 | 29.9930 | 0.00775 | 401.98 | 0.479 | +25.66 | 31.43 |
| N8 | 69.99975 | 29.9925 | 0.00775 | 226.80 | 0.417 | +23.64 | 30.83 |
| N9 | 69.99925 | 29.9975 | 0.00325 | 105.38 | 0.279 | +18.38 | 21.34 |

To evaluate the response of the formulations when replacing BSA with PspA1, PspA4Pro or a mixture of the two, maintaining the concentration at 0.3 mg/mL of total protein, an assay was performed with each of the combinations. Table 2 brings a summary of the combinations studied for formulations A and B.

**Table 2 - Composition of LPs A and B formulations containing PspA.**

| Formulation | Total lipids (mg) | DPPC (mg/mL) | DC - Chol (mg/mL) | αGalCer (µg/mL) | PspA1 (mg/mL) | PspA4 Pro (mg/m L) |
|---|---|---|---|---|---|---|
| LP - DPPC:DC-Chol empty (1A) | 15.00 | 11.70 | 3.30 | - | - | - |
| LP - DPPC:DC-Chol PspA1 (2A) | 15.00 | 11.70 | 3.30 | - | 0.30 | - |
| LP - DPPC:DC-Chol PspA4Pro (3A) | 15.00 | 11.70 | 3.30 | - | - | 0.30 |
| LP - DPPC:DC-Chol PspA1+PspA4Pro (4A) | 15.00 | 11.70 | 3.30 | - | 0.15 | 0.15 |
| LP - DPPC:DC-Chol:αGalCer empty (1B) | 15.00 | 11.60 | 3.30 | 0.13 | - | - |
| LP - DPPC:DC-Chol:αGalCer PspA1 (2B) | 15.00 | 11.60 | 3.30 | 0.13 | 0.30 | - |
| LP - DPPC:DC-Chol:αGalCer PspA4Pro (3B) | 15.00 | 11.60 | 3.30 | 0.13 | - | 0.30 |
| LP - DPPC:DC-Chol:αGalCer PspA1+PspA4Pro (4B) | 15.00 | 11.60 | 3.30 | 0.13 | 0.15 | 0.15 |

After producing the formulations in duplicate, following the necessary specificities, samples were collected to characterize the size, PdI and charge of the particles, together with the encapsulation efficiency (EE(%)). Table 3 contains the means of these results and the respective standard deviations.

**Table 3 - Characterization of the liposomes of the formulations containing PspA1 and PspA4Pro.**

| Formulation | Size (nm) | PdI | Charge (mV) | EE (%) |
|---|---|---|---|---|
| LP - DPPC:DC-Chol empty (1A) | 557.90 ± 207.25 | 0.423 ± 0.065 | +34.80 ± 9.04 | - |
| LP - DPPC:DC-Chol PspA1 (2A) | 114.46 ± 22.48 | 0.178 ± 0.041 | +38.67 ± 3.15 | 99.57 ± 0.58 |
| LP - DPPC:DC-Chol PspA4Pro (3A) | 109.25 ± 6.58 | 0.261 ± 0.103 | +45.03 ± 3.58 | 88.74 ± 17.85 |
| LP - DPPC:DC-Chol PspA1+PspA4Pro (4A) | 135.17 ± 41.52 | 0.240 ± 0.017 | +40.07 ± 3.36 | 99.43 ± 0.99 |
| LP - DPPC:DC-Chol:αGalCer empty (1B) | 646.41 ± 192.73 | 0.462 ± 0.032 | +34.59 ± 15.52 | - |
| LP - DPPC:DC-Chol:αGalCer PspA1 (2B) | 125.88 ± 38.98 | 0.170 ± 0.026 | +38.66 ± 2.89 | 99.99 ± 0.01 |
| LP - DPPC:DC-Chol:αGalCer PspA4Pro (3B) | 113.46 ± 20.20 | 0.259 ± 0.054 | +42.69 ± 1.94 | 75.04 ± 19.34 |
| LP - DPPC:DC-Chol:αGalCer PspA1+PspA4Pro (4B) | 160.02 ± 64.12 | 0.194 ± 0.051 | +39.62 ± 1.57 | 98.53 ± 2.55 |

Therefore, after preparation, the different formulations containing PspA4Pro were used in mouse immunization experiments. Formulation B, composed of DPPC:DC-Chol:aGalCer, was chosen for inducing the highest titers of anti-PspA4Pro serum antibodies. Then, experiments were carried out with formulations B containing PspA1, PspA4Pro and a combination of the two proteins. A high induction of antibodies against both proteins and protection against two pneumococcal strains expressing PspA from different families was demonstrated.

### - Production of nanocomposite microparticle carriers (NCMPs) :

NCMPs incorporating LPs containing an encapsulated protein or mixture of proteins were prepared by an adapted method of SD previously described (KUNDA et al., 2015) . After centrifugation of the LPs produced and recovered from the NanoAssemblr^{®}, the precipitates were resuspended in an aqueous solution of a sugar (trehalose, mannitol, sucrose or lactose) supplemented or not by L-leucine, using a ratio of LPs/sugar/L-leucine of 1:20:0.5 (m/m/m). The Büchi B-290 mini spray-dryer (*Büchi Labortechnik)* was adjusted to obtain an inlet temperature of 90°C and 45-50°C as an outlet temperature. The machine operated with an airflow of 670 L/h, aspirator at 40 L/h and sample feed rate at 1.5 mL/min. The particles were collected in tubes with screw closure and stored in a desiccator at room temperature.

For the preferred formulation, the ideal excipient for drying the LPs was chosen following the order of importance of smaller size, lower PdI and higher yield, to select the one that facilitates the redispersion of the LPs in aqueous solution and presents good performance of the process. Trehalose without L-leucine supplementation was then defined as the ideal excipient for the SD process of LPs.

The drying process for the formation of NCMPs was kept unchanged due to the good results obtained. Thus, the LPs were resuspended in a trehalose solution, obeying a proportion of 20 mg of trehalose for each 1 mg of lipid, and taken to SD following the same parameters previously used. The powders obtained were stored in a desiccator at room temperature until characterization. The dried samples were resuspended in deionized water, homogenized for 30 seconds by sonication and diluted again in water to perform the analysis. Table 4 presents the results of the characterization after spray dry.

**Table 4 - Characterization of LPs released from NCMPs after resuspension in water.**

| Formulation | Size (nm) | PdI | Charge (mV) |
|---|---|---|---|
| LP/NCMP - DPPC:DC-Chol empty (1A) | 670.18 ± 348.17 | 0.564 ± 0.564 | +10.14 ± 4.83 |
| LP/NCMP - DPPC:DC-Chol PspA1 (2A) | 147.44 ± 28.31 | 0.209 ± 0.209 | +39.03 ± 1.40 |
| LP/NCMP - DPPC:DC-Chol PspA4Pro (3A) | 218.07 ± 20.06 | 0.286 ± 0.286 | +35.68 ± 4.88 |
| LP/NCMP - DPPC:DC-Chol PspA1+PspA4Pro (4A) | 167.94 ± 12.34 | 0.256 ± 0.176 | +43.14 ± 3.58 |
| LP/NCMP - DPPC:DC-Chol:αGalCer empty (1B) | 366.13 ± 96.76 | 0.308 ± 0.176 | +16.93 ± 12.62 |
| LP/NCMP - DPPC:DC-Chol:αGalCer PspA1 (2B) | 172.27 ± 12.71 | 0.239 ± 0.163 | +39.45 ± 2.81 |
| LP/NCMP - DPPC:DC-Chol:αGalCer PspA4Pro (3B) | 205.93 ± 40.65 | 0.250 ± 0.032 | +37.50 ± 4.24 |
| LP/NCMP - DPPC:DC-Chol:αGalCer PspA1+PspA4Pro (4B) | 175.79 ± 33.14 | 0.155 ± 0.171 | +39.95 ± 3.61 |

The yield (Y (%)) of the process, the remaining moisture (M (%)) in the formulations were also determined. Finally, the protein concentration was estimated by LP/NCMP in mass (PC mg/mg). Table 5 presents the results obtained by the described calculations.

**Table 5 - Evaluation of process yield, remaining moisture and protein concentration after drying.**

| Formulation | Y (%) | M (%) | PC powder mg/mg | |
|---|---|---|---|---|
| | | | PspA1 | PspA4Pro |
| LP/NCMP - DPPC:DC-Chol empty (1A) | 62.44 ± 9.47 | 4.89 ± 0.34 | - | - |
| LP/NCMP - DPPC:DC-Chol PspA1 (2A) | 73.30 ± 3.89 | 4.32 ± 0.60 | 0.010 | - |
| LP/NCMP - DPPC:DC-Chol PspA4Pro (3A) | 67.08 ± 3.51 | 6.21 ± 2.39 | - | 0.010 |
| LP/NCMP - DPPC:DC-Chol PspA1+PspA4Pro (4A) | 78.13 ± 5.36 | 4.47 ± 0.59 | 0.005 | 0.005 |
| LP/NCMP - DPPC:DC-Chol:αGalCer empty (1B) | 66.75 ± 5.59 | 4.29 ± 0.53 | - | - |
| LP/NCMP - DPPC:DC-Chol:αGalCer PspA1 (2B) | 75.75 ± 6.81 | 4.47 ± 0.54 | 0.010 | - |
| LP/NCMP - DPPC:DC-Chol:αGalCer PspA4Pro (3B) | 66.72 ± 2.17 | 4.33 ± 0.60 | - | 0.010 |
| LP/NCMP - DPPC:DC-Chol:αGalCer PspA1+PspA4Pro (4B) | 76.67 ± 3.27 | 4.54 ± 0.19 | 0.005 | 0.005 |

The NCMPs were also morphologically characterized by SEM and the captures of the samples obtained are gathered and arranged in Figure 2.

For the evaluation of the preservation of the biological activity and integrity of the proteins encapsulated by the LPs and formulated in NCMPs, assays of recognition by specific antibodies and binding of lactoferrin to the proteins released from the samples after lysis of the particles were carried out. For the *western blot,* about 250 ng of total protein from the LP/NCMPs was applied to 12% SDS-PAGE along with different amounts of PspA1 and PspA4Pro. After transfer to nitrocellulose membrane, incubation with anti-PspA1 or anti-PspA4Pro polyclonal serum, followed by anti-mouse IgG conjugated to peroxidase enzyme and detection by chemiluminescence, images of the membranes were obtained (Figure 3).

It is shown in Figure 3 that after disrupting the particles and releasing the proteins, samples were separated on 12% SDS-PAGE. PspA1 (A and C) and PspA4Pro (B and D) were added as controls in amounts of 500 (1), 250 (2), 100 (3), 50 (4) and 10 (5) ng. Particles of DPPC:DC-Chol (A and B) and DPPC:DC-Chol:aGalCer (C and D) were applied in order to contain, in the channel, 250 ng of PspA1 (8A, 8B, 7C and 7D), PspA4Pro (7A, 7B, 8C and 8D) or the PspA1+PspA4Pro combination (9A, 9B, 9C and 9D) . An amount of empty particle sample (6A, 6B, 6C and 6D) equivalent to the largest applied volume was added. After transfer, the membranes were incubated with anti-PspA1 (A and C) (1:5000) or anti-PspA4Pro (B and C) (1:5000) polyclonal serum, followed by incubation with anti-mouse IgG conjugated to peroxidase (1:1000) and detection by chemiluminescence. Arrows represent the expected size of PspA1 and PspA4Pro.

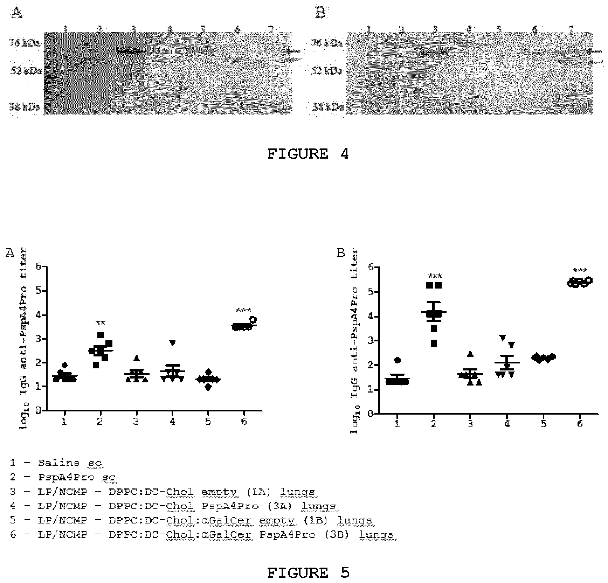

For the lactoferrin binding assay, the equivalent of 2 µg of total protein, recovered after particle disruption, was applied along with BSA, PspA1 and PspA4Pro in 12% SDS-PAGE. After transfer to nitrocellulose membrane, blocking, incubation with lactoferrin conjugated to biotin and streptavidin conjugated to peroxidase enzyme, the bands were detected by chemiluminescence and the images obtained were gathered and displayed in Figure 4.

In Figure 4 it is shown that after the disruption of the particles and release of the proteins, the samples were separated on 12% SDS-PAGE. 2 µg of BSA (1A and 1B), PspA1 (2A and 2B) and PspA4Pro (3A and 3B) were added as a control. Particles of DPPC:DC-Chol (A) and DPPC:DC-Chol:αGalCer (B) were applied in order to contain, in the well, 2 µg of PspA1 (6A and 5B), PspA4Pro (5A and 6B) or the combination PspA1+PspA4Pro (7A and 7B). An amount of empty particle sample (4A and 4B) equivalent to the largest applied volume was added. After transfer, the membranes were incubated with lactoferrin conjugated to biotin at a concentration of 4 pg/mL, followed by incubation with streptavidin conjugated to peroxidase (1:100). Lower and upper arrows represent the expected size of PspA1 and PspA4Pro, respectively.

Thus, the production of LPs with high encapsulation efficiency of PspA1 and PspA4Pro was demonstrated, in particles of size around 100 nm and PdI close to 0.2 for formulations A and B. These particles did not change during the drying process, since similar values for size, PdI and surface charge after resuspension of the powders in water were obtained. SEM analysis also showed particles with homogeneous morphology. The formulations also kept the PspA1 and PspA4Pro proteins intact, with maintenance of specific serum recognition and binding to lactoferrin.

### - Evaluation of the immunogenicity of LP/NCMP formulations containing PspA:

After the production and confirmation of the stability of the formulations, the evaluation of the immune response induced by the different particles developed was carried out, initially using PspA4Pro as a reference to reduce the number of animals in the experiment.

In this step, female BALB/c mice were divided into 6 groups containing 6 animals. As a control, one group received saline solution and another was immunized with 6 µg of PspA4Pro, both subcutaneously. The other 6 groups were anesthetized and immunized with resuspension of LP/NCMPs in saline, in a powder concentration equivalent to 6 µg of total protein. Therefore, one group received a resuspension of 12.0 pg/pL 3A - LP/NCMP - DPPC:DC-Chol PspA4Pro (0.60 mg total per animal), the other received a resuspension of 12.0 pg/pL 3B - LP/NCMP - DPPC:DC-Chol:αGalCer PspA4Pro (0.60 mg total per animal. The groups that received 1A - LP/NCMP-DPPC:DC-Chol empty and 1B - LP/NCMP - DPPC:DC-Chol:αGalCer empty were immunized with the same mass of powder by pulmonary instillation, equivalent to the mass used in the groups immunized with LP/NCMPs containing protein. The immunizations were administered in 2 doses with an interval of 15 days between each dose. Blood samples were collected 14 days after each dose to determine the animals' anti-PspA4Pro IgG antibody titer by ELISA. Figure 5 shows graphs with antibody titers of individual mice after one (A) and two (B) doses.

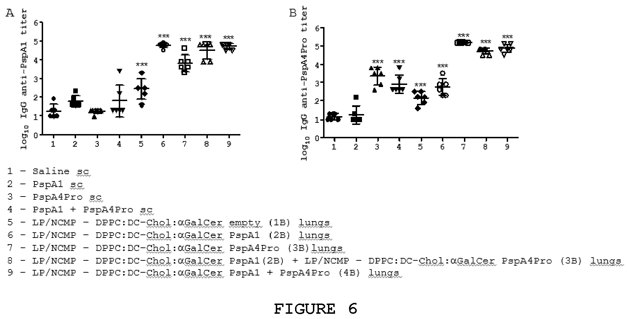

Due to the promising results obtained in the induction of serum IgG antibodies, Formulation B, composed of DPPC:DC-Chol:aGalCer, was selected to evaluate the immune response with PspA1, PspA4Pro proteins or a combination of both. For this, female BALB/c mice were divided into 9 groups containing 6 animals each. As controls, one group received 100 µL of saline subcutaneously and the other 3 groups were immunized, also subcutaneously, with 100 µL of saline solution containing 6 µg of PspA1, 6 µg PspA4Pro or a mixture containing 3 µg of PspA1 and 3 µg PspA4Pro. The other 5 groups were anesthetized and immunized, via instillation, with a resuspension of LP/NCMPs containing the equivalent of 6 µg of total protein in each dose. Thus, one group received a resuspension of 12.0 pg/pL 2B - LP/NCMP - DPPC:DC-Chol:αGalCer PspA1 (0.60 mg total per animal), the other received 12.0 pg/pL 3B - LP/NCMP - DPPC:DC-Chol:αGalCer PspA4Pro (0.60 mg total per animal), another the resuspension of 12.0 pg/pL 4B - LP/NCMP - DPPC:DC-Chol:αGalCer PspA1+PspA4Pro (0.60 mg total per animal) and one group received a mixture of 6.0 pg/pL 2B - LP/NCMP - DPPC:DC-Chol:αGalCer PspA1 (0.30 mg total per animal) and 6.0 pg/pL 3B - LP/NCMP - DPPC:DC-Chol:αGalCer PspA4Pro (0.30 mg total per animal). One group received a resuspension of 12.0 pg/pL 1B - LP/NCMP - DPPC:DC-Chol:αGalCer empty (0.60 mg total per animal) by pulmonary instillation, equivalent to the mass used in groups immunized with protein-containing LP/NCMPs. The immunizations were administered in 2 doses with an interval of 15 days between each dose. Blood samples were collected 14 days after each dose to determine the anti-PspA1 and anti-PspA4Pro IgG antibody titers of the animals. The induction of antibodies against PspA1 and PspA4Pro in the animals' serum was determined by ELISA assay. Figure 6 shows the graphs with the titers of the animals after two doses and indicate the induction of high titers of antibodies by the new pulmonary formulations.

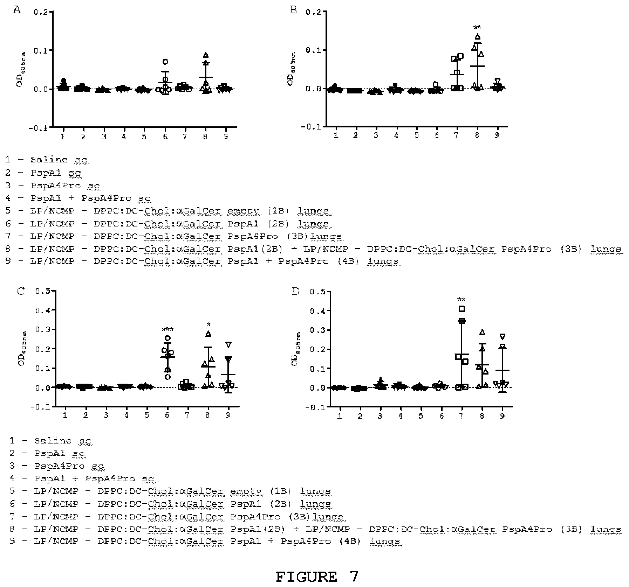

On the 14th day after the second dose and in the 4 days that followed, the vaginal wash was collected from all animals. At the end of the 5th day, the samples belonging to the same animal were mixed and an ELISA assay was performed to determine IgA and IgG antibodies in the mucosa of the animals. In Figure 7 it is possible to observe the graphs of the specific antibodies detected in the mucosa of the immunized animals.

The group immunized with 2B - LP/NCMP-DPPC:DC-Chol:αGalCer PspA1 showed statistical difference only in the induction of mucosal anti-PspA1 IgG, with a tendency to increase anti-PspA1 IgA. The group immunized with 3B - LP/NCMP - DPPC:DC-Chol:αGalCer PspA4Pro also only showed a statistical difference in the induction of anti-PspA4Pro IgG and a tendency towards an increase in anti-PspA4Pro IgA. The group immunized with the mixture 2B - LP/NCMP - DPPC:DC-Chol:αGalCer PspA1 + 3B - LP/NCMP - DPPC:DC-Chol:αGalCer PspA4Pro showed a significant increase in the induction of anti-PspA1 IgG and anti-PspA4Pro IgA, with a tendency towards an increase in anti-PspA1 IgA and anti-PspA4Pro IgG. However, the group immunized with 4B - LP/NCMP - DPPC:DC-Chol:αGalCer PspA1+PspA4Pro did not show significant induction, only a tendency to increase IgG against the two proteins.

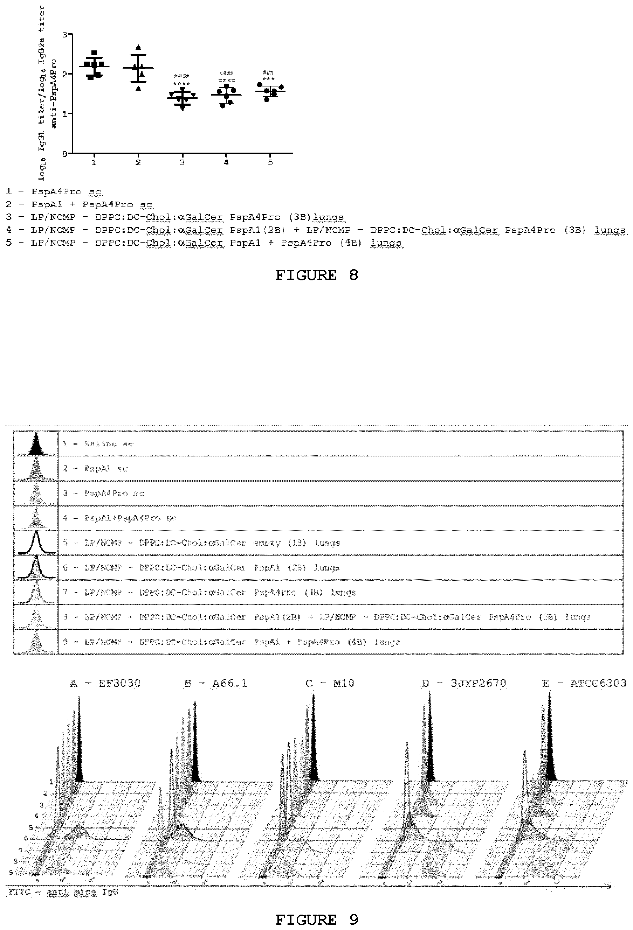

To assess the type of humoral response induced, serum IgG1 and IgG2a antibodies were measured. Only anti-PspA4Pro antibodies were evaluated, which showed higher titers of total IgG. After determining the titer, the ratio between IgG1 and IgG2a was determined, as shown in Figure 8.

The groups immunized with the proteins only subcutaneously induced higher IgG1 titers, since the ratio between the titers of the two isotypes tend to values above 2. The groups immunized with 3B - LP/NCMP - DPPC:DC-Chol:αGalCer PspA4Pro, the 2B - LP/NCMP - DPPC:DC-Chol:αGalCer PspA1 + 3B - LP/NCMP - DPPC:DC-Chol:αGalCer PspA4Pro and 4B - LP/NCMP - DPPC:DC-Chol:αGalCer PspA1+PspA4Pro showed a more balanced response, close to 1.

Next, the binding capacity of serum antibodies to the surface of different strains of pneumococcus was evaluated. The strains EF3030 (serotype 19F, PspA1), A66.1 (serotype 3, PspA2), M10 (serotype 11A, PspA3), 3JYP2670 (serotype 3, PspA4) and ATCC6303 (serotype 3, PspA5) were grown and incubated with the mixture of sera from animals in each group. Figure 9 shows the graphs of binding to the different strains and in Table 6 it is possible to observe the median fluorescence intensity (MFI) of each group.

**Table 6 - MFI of samples with pneumococcal strains incubated with serum from animals immunized with LP/NCMPs containing PspA.**

| Group | EF3030 | A66.1 | M10 | 3YJP2670 | ATCC 6303 |
|---|---|---|---|---|---|
| Saline sc | 16.1 | 20.3 | 33.0 | 21.7 | 44.2 |
| PspA1 sc | 20.3 | 23.1 | 31.5 | 24.5 | 44.2 |
| PspA4Pro sc | 17.5 | 18.9 | 74.5 | 258.0 | 190.0 |
| PspA1+PspA4Pro sc | 16.1 | 27.3 | 47.0 | 294.0 | 169.0 |
| LP/NCMP - DPPC:DC-Chol:αGalCer empty (1B) lungs | 23.1 | 37.2 | 35.8 | 40.0 | 59.7 |
| LP/NCMP - DPPC:DC-Chol:αGalCer PspA1 (2B) lungs | 1128.0 | 564.0 | 33.0 | 346.0 | 359.0 |
| LP/NCMP - DPPC:DC-Chol:αGalCer PspA4Pro (3B) lungs | 783.0 | 61.1 | 853.0 | 5613.0 | 4848.0 |
| LP/NCMP - DPPC:DC-Chol:αGalCer PspA1 (2B) + LP/NCMP - DPPC:DC-Chol:αGalCer PspA4Pro (3B) lungs | 704.0 | 673.0 | 339.0 | 4424.0 | 4677.0 |
| LP/NCMP - DPPC:DC-Chol:αGalCer PspA1+PspA4Pro (4B) lungs | 765.0 | 447.0 | 769.0 | 5647.0 | 3764.0 |

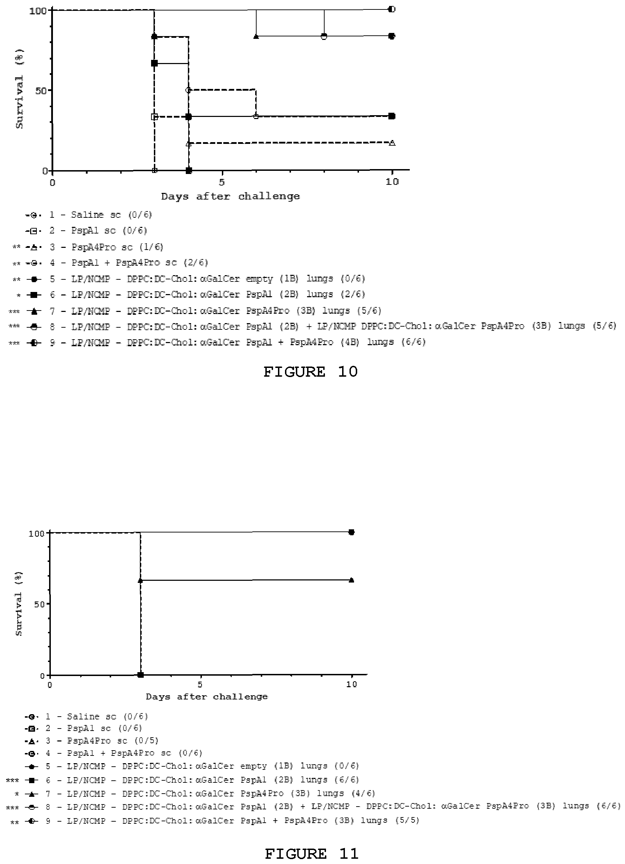

A broad binding to the surface of different strains by antibodies induced by the mixture 2B - LP/NCMP - DPPC:DC-Chol:αGalCer PspA1 + 3B - LP/NCMP - DPPC:DC-Chol:αGalCer PspA4Pro and by the formulation 4B - LP/NCMP - DPPC:DC-Chol:αGalCer PspA1+PspA4Pro was observed. The 3B - LP/NCMP - DPPC:DC-Chol:αGalCer PspA4Pro formulation was able to induce antibodies that efficiently bind to strains expressing PspA from family 2 and to at least one strain expressing PspA from family 1. Then, the animals were challenged with a virulent strain of pneumococcus to evaluate the protection induced by the formulations against the bacteria. Anesthetized mice received a suspension of *S. pneumoniae* strain ATCC6303 (serotype 3, PspA5, family 2) intranasally targeting the lungs and were followed up for 10 days. Figure 10 shows the survival time curve of the groups and Table 7 shows the final survival results.

**Table 7 - Final survival of mice immunized with LP/NCMPs containing PspA1 and PspA4Pro after challenge with the ATCC6303 strain (serotype 3, PspA5, family 2) and analysis by Fisher's Exact Test.**

| Group | Survival (%) | P-value | Significance |
|---|---|---|---|
| PspA1 sc | 0 | 1.0000 | ns |
| PspA4Pro sc | 17 | 1.0000 | ns |
| PspA1+PspA4Pro sc | 33 | 0.4545 | ns |
| LP/NCMP - DPPC:DC-Chol:aGalCer empty (1B) lungs | 0 | 1.0000 | ns |
| LP/NCMP - DPPC:DC-Chol:aGalCer PspA1 (2B) lungs | 33 | 0.4545 | ns |
| LP/NCMP - DPPC:DC-Chol:aGalCer PspA4Pro (3B) lungs | 83 | 0.0152 | * |
| LP/NCMP - DPPC:DC-Chol:aGalCer PspA1 (2B) + LP/NCMP - DPPC:DC-Chol:aGalCer PspA4Pro (3B) lungs | 83 | 0.0152 | * |
| LP/NCMP - DPPC:DC-Chol:aGalCer PspA1+PspA4Pro (4B) lungs | 100 | 0.0022 | ** |

Between the tested formulations, only 4B - LP/NCMP - DPPC:DC-Chol:αGalCer PspA1+PspA4Pro showed 100% survival. The groups immunized with the formulation 3B - LP/NCMP - DPPC:DC-Chol:αGalCer PspA4Pro and the mixture 2B - LP/NCMP - DPPC:DC-Chol:αGalCer PspA1 + 3B - LP/NCMP - DPPC:DC-Chol:αGalCer PspA4Pro showed a survival rate of approximately 83%. By Fisher's Exact Test, only these 3 groups showed statistical difference with the saline group.

To assess the breadth of protection, a new challenge was performed with a strain that expresses PspA from family 1. A similar number of animals were distributed into 9 groups and received the same immunization schedule. The animals were challenged intranasally targeting the lungs with the virulent strain A66.1 (serotype 3, PspA2, family 1) of *S. pneumoniae* after 21 days of the second dose and followed for 10 days. Figure 11 presents the survival time curve of the groups and Table 8 shows the results after the final survival analysis.

**Table 8 - Final survival of mice immunized with LP/NCMPs containing PspA1 and PspA4Pro after challenge with the A66.1 strain (serotype 3, PspA2, family 1) and analysis by Fisher's Exact Test.**

| Group | Survival (%) | P-value | Significance |
|---|---|---|---|
| PspA1 sc | 0 | 1.0000 | ns |
| PspA4Pro sc | 0 | 1.0000 | ns |
| PspA1+PspA4Pro sc | 0 | 1.0000 | ns |
| LP/NCMP - DPPC:DC-Chol:aGalCer empty (1B) lungs | 0 | 1.0000 | ns |
| LP/NCMP - DPPC:DC-Chol:aGalCer PspA1 (2B) lungs | 100 | 0.0022 | ** |
| LP/NCMP - DPPC:DC-Chol:aGalCer PspA4Pro (3B) lungs | 67 | 0.0606 | ns |
| LP/NCMP - DPPC:DC-Chol:aGalCer PspA1 (2B) + LP/NCMP-DPPC:DC-Chol:aGalCer PspA4Pro (3B) lungs | 100 | 0.0022 | ** |
| LP/NCMP - DPPC:DC-Chol:aGalCer PspA1+PspA4Pro (4B) lungs | 100 | 0.0022 | ** |

Against the A66.1 strain, the survival of the group immunized with 3B - LP/NCMP - DPPC:DC-Chol:αGalCer PspA4Pro was approximately 67%, but without statistical significance, according to Fisher's Exact Test. However, confirming the result of the previous experiment, the group immunized with 4B - LP/NCMP - DPPC:DC-Chol:αGalCer PspA1+PspA4Pro presented a survival of 100% with significance by Fisher's Test. In this experiment, two other groups showed 100% survival and also statistical significance by Fisher's Exact Test, animals immunized with the formulation 2B - LP/NCMP - DPPC:DC-Chol:αGalCer PspA1 and the mixture 2B - LP/NCMP - DPPC:DC-Chol:αGalCer PspA1 + 3B - LP/NCMP - DPPC:DC-Chol:αGalCer PspA4Pro.

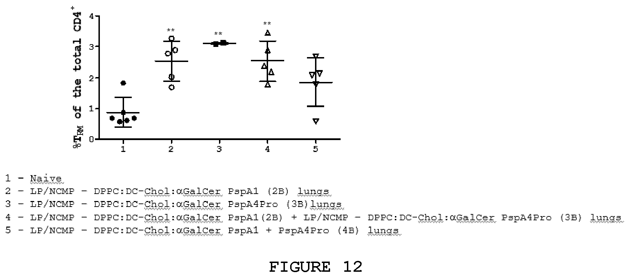

After observing the survival of the animals, it was evaluated whether the tested formulation would have the ability to induce a cellular memory response in the lung tissue. For this purpose, approximately 2 weeks after the end of the challenge experiment, the lungs of the animals that survived the challenge with the A66.1 pneumococcus strain were extracted, minced and stained with a panel containing a combination of monoclonal antibodies for CD4+ resident memory T cells (TRM) . As a control, a group of 6 neither immunized nor challenged (naive) animals was added to the analysis. After analyzing the data to determine the TRM population (CD45+CD4+CD11aHighCD69+CD44HighCD62LLow), the percentage of TRMs was calculated in relation to the total number of CD4+ cells (Figure 12).

Among the surviving groups, it was possible to observe a significant increase in TRM in 3 of the 4 remaining groups of the challenge experiment. Only the group immunized with 4B - LP/NCMP - DPPC:DC-Chol:αGalCer PspA1+PspA4Pro did not show statistical difference with the naive group, however it is possible to observe a clear tendency to increase.

In conclusion, the present invention proposes formulations that were very promising, inducing high titers of serum IgG antibodies against PspA from family 1 and family 2. In addition, protection was demonstrated against challenge with pneumococci expressing the 2 families of PspA most commonly expressed in clinical isolates. Thus, it was possible to present the formulation of the present invention as a vaccine candidate with protection against pneumococcus.

In summary, the invention refers to the following aspects, as defined in the following numbered items:
1. Process for obtaining liposomal particles comprising at least one pneumococcal surface protein A (PspA) and an adjuvant molecule formulated in nanocomposite microparticle carriers (NCMP), wherein the process comprises the following steps:
   a.1) Mixing from 50 to 90% in moles of dipalmitoylphosphatidylcholine (DPPC), and from 10 to 50% in moles of 3β-[N-(N',N'-dimethylaminoethane)-carbamoyl]cholesterol (DC-Col), in ethanol to organic phase;
   a.2) Adding from 0.00100 to 0.00775% in moles of an adjuvant molecule consisting of αGalCer, in said mixture obtained in step "a.1";
   a.3) Adding 10 to 20 mg/mL of said mixture obtained in step "a.2", in organic phase and aqueous solution, which contains a concentration of 0.3 to 2.0 mg/mL of a solution of one or more PspA;
      a) Centrifuging the LPs obtained in step "a" to remove non-encapsulated protein;
      b) Resuspending said LPs in a sugar solution;
      c) Drying the solution obtained in step "c" by atomization spray-drying (SD) to obtain at the end a powder containing NCMPs carrying the LPs.
2. Process, according to claim 1, **characterized in that** the substep "a.1)" preferably mixing 69.99900% in moles of DPPC; and 30.00000% in moles of DC-Chol in 1 ml ethanol to organic phase; the substep "a.2" preferably adding 0.00100% in moles of αGalCer in said mixture obtained in step "a.1"; the substep "a.3" preferably adding 15 mg/mL of said mixture obtained in step "a.2", preferably 1 mL of organic phase to 5 ml of aqueous solution, which contains a concentration of preferably 0.3 mg/mL of a solution of one or more PspA.
3. Process, according to item 1 or 2, wherein the step "a" is performed by microfluidic technique.
4. Process, according to any one of items 1-3, wherein, in step "a", PspA is selected from the group consisting of PspA1 (GenBank AY082387), PspA4Pro (GenBank EF649969.1), or a mixture thereof, wherein preferably PspA1 consists of the amino acid sequence as set out in SEQ ID NO:1, which is translated by the PspA1 gene sequence as set out in SEQ ID NO:2; and PspA4Pro consists of the amino acid sequence as set forth in SEQ ID NO:3, which is translated by the PspA4Pro gene sequence as set out in SEQ ID NO:4, wherein more preferably PspA solution comprises from 0.3 to 2.0 mg/mL of PspA1, preferably 0.3 mg/mL; or from 0.3 to 2.0 mg/mL of PspA4Pro, preferably 0.3 mg/mL; or from 0.3 to 2.0 mg/mL of 1:1 (PspA1:PspA4Pro), preferably 0.3 mg/mL.
5. Process, according to any one of items 1-4, wherein, in step "a", the aqueous solution is composed of deionized water or a solution of lyophilized PspA1, or PspA4Pro or a mixture of PspA1 and PspA4Pro resuspended in water, dialyzed in PBS and diluted in deionized water, preferably at a concentration of 0.3 mg/mL.
6. Process, according to any one of items 1-5, wherein in step "b" centrifugation is, preferably at 84,035 g for 35 minutes at a temperature from 18 to 25 °C; and in step "c", the sugar is selected from the group consisting of trehalose, mannitol, sucrose or lactose, wherein preferably the sugar is used in a ratio of 20:1 (sugar:lipid), and more preferably the sugar is trehalose; and after step "d" of SD, the yield of the drying process, for the formulation obtained, was between 60 and 70%, resulting in particles with a final moisture content between 4 and 5%.
7. Liposomal particles (LP) obtained from the process as defined in any one of items 1 to 6, wherein said LP/NCMPs comprises:
   - from 50 to 90% in moles of dipalmitoylphosphatidylcholine (DPPC), and from 10 to 50% in moles of 3β-[N-(N',N'-dimethylaminoethane)-carbamoyl]cholesterol (DC-Col), and from 0.00100 to 0 .00775% in moles of an adjuvant molecule consisting of αGalCer forming the organic phase;
   - from 0.3/mL to 2.0 mg/mL of one or more PspA in aqueous solution; and
   - sugar.
8. Liposomal particles, according to item 7, wherein preferably comprises:
   - 69.99900% moles of DPPC, 30.00000% moles of DC-Chol, and 0.00100% moles of αGalCer forming the organic phase; and
   - 0.3 mg/mL of PspA1; or 0.3 mg/mL of PspA4Pro; or 0.3 mg/mL of 1:1 (PspA1:PspA4Pro).
9. Liposomal particles, according to item 7 or 8, wherein PspA is selected from the group consisting of PspA1 (GenBank AY082387), PspA4Pro (GenBank EF649969.1), or a mixture thereof, wherein preferably, PspA1 consists of the amino acid sequence as set out in SEQ ID NO:1, which is translated by the PspA1 gene sequence as set out in SEQ ID NO:2; and PspA4Pro consists of the amino acid sequence as set forth in SEQ ID NO:3, which is translated by the PspA4Pro gene sequence as set out in SEQ ID NO:4.
10. Liposomal particles, according to any one of items 7-9, wherein preferably comprise a sugar in the ratio of 20:1 (sugar:lipid), wherein the sugar is selected from the group consisting of trehalose, mannitol, sucrose or lactose, preferably trehalose.
11. Liposomal particles, according to any one of items 7-10, wherein the LP/NCMPs:
   - are in powder format, dried by atomization spray-drying (SD);
   - have average size ranges from 172 to 366 nm, preferably 172 nm;
   - have PdI ranges from 0.155 to 0.308, preferably 0.200;
   - have zeta potential ranges from +16.93 to +39.95, preferably +38.00; and
   - have encapsulation efficiency ranges from 75.04 to 99.99%, preferably 90.00%.
12. Liposomal particles, according to any one of items 7-11, wherein are for use as a vaccine with comprehensive protection against pneumococcus expressing PspA from family 1 and family 2.
13. Use of LP/NCMPs as defined in any one of items 7-12, wherein it is for the preparation of an immunogenic composition for comprehensive protection against pneumococcus expressing PspA from family 1 and family 2 , wherein the immunogenic composition is preferably a vaccine powder for mucosal administration.
14. Immunogenic composition wherein it comprises the LP/NCMPs as defined in any one of items 7-12, and a pharmaceutically acceptable carrier and/or adjuvant, wherein the immunogenic composition is preferably a vaccine powder, more preferably to be administered via mucosal route, more preferably targeting the lungs.
15. Immunogenic composition, according to item 14, wherein it is for use as a vaccine with comprehensive protection against pneumococcus expressing PspA from family 1 and family 2.

Thus, the embodiments presented in the present disclosure do not limit the totality of possibilities, and it will be understood that various omissions, substitutions and changes can be made by a person skilled in the art, without departing from the scope of the present invention.

It is understood that all combinations of the elements that perform the same function or substantially use the same ways to achieve similar results are within the scope of this invention. Substitutions of elements from one described embodiment to another are also fully intended and contemplated.

A person skilled in the art will value the knowledge presented here and will be able to reproduce the invention in the presented embodiments and in other variants, covered in the scope of the claims.

### BIBLIOGRAPHIC REFERENCES

KUNDA, N. K. et al. Pulmonary Dry Powder Vaccine of Pneumococcal Antigen Loaded Nanoparticles. International Journal of Pharmaceutics, v. 495, n. 2, p. 903-912, 2015.

## Claims

1. Process for obtaining liposomal particles comprising at least one pneumococcal surface protein A (PspA) and an adjuvant molecule formulated in nanocomposite microparticle carriers (NCMP) **characterized in that** the process comprises the following steps:
d) Preparing liposomal particles (LPs) from the following substeps:
a.1) Mixing from 50 to 90% in moles of dipalmitoylphosphatidylcholine (DPPC), and from 10 to 50% in moles of 3β-[N-(N',N'-dimethylaminoethane)-carbamoyl]cholesterol (DC-Col), in ethanol to organic phase;
a.2) Adding from 0.00100 to 0.00775% in moles of an adjuvant molecule consisting of αGalCer, in said mixture obtained in step "a.1";
a.3) Adding 10 to 20 mg/mL of said mixture obtained in step "a.2", in organic phase and aqueous solution, which contains a concentration of 0.3 to 2.0 mg/mL of a solution of one or more PspA;
e) Centrifuging the LPs obtained in step "a" to remove non-encapsulated protein;
f) Resuspending said LPs in a sugar solution;
g) Drying the solution obtained in step "c" by atomization spray-drying (SD) to obtain at the end a powder containing NCMPs carrying the LPs.

2. Process, according to claim 1, **characterized in that** the substep "a.1)" preferably mixing 69.99900% in moles of DPPC; and 30.00000% in moles of DC-Chol in 1 ml ethanol to organic phase; the substep "a.2" preferably adding 0.00100% in moles of αGalCer in said mixture obtained in step "a.1"; the substep "a.3" preferably adding 15 mg/mL of said mixture obtained in step "a.2", preferably 1 mL of organic phase to 5 ml of aqueous solution, which contains a concentration of preferably 0.3 mg/mL of a solution of one or more PspA.

3. Process, according to claim 1 or 2, **characterized in that** the step "a" is performed by microfluidic technique.

4. Process, according to any one of claims 1-3, **characterized in that,** in step "a", PspA is selected from the group consisting of PspA1 (GenBank AY082387), PspA4Pro (GenBank EF649969.1), or a mixture thereof, wherein preferably PspA1 consists of the amino acid sequence as set out in SEQ ID NO:1, which is translated by the PspA1 gene sequence as set out in SEQ ID NO:2; and PspA4Pro consists of the amino acid sequence as set forth in SEQ ID NO:3, which is translated by the PspA4Pro gene sequence as set out in SEQ ID NO:4, wherein more preferably PspA solution comprises from 0.3 to 2.0 mg/mL of PspA1, preferably 0.3 mg/mL; or from 0.3 to 2.0 mg/mL of PspA4Pro, preferably 0.3 mg/mL; or from 0.3 to 2.0 mg/mL of 1:1 (PspA1: PspA4Pro), preferably 0.3 mg/mL.

5. Process, according to any one of claims 1-4, **characterized in that,** in step "a", the aqueous solution is composed of deionized water or a solution of lyophilized PspA1, or PspA4Pro or a mixture of PspA1 and PspA4Pro resuspended in water, preferably dialyzed in PBS and diluted in deionized water at a concentration of 0.3 mg/mL.

6. Process, according to any one of claims 1-5, **characterized in that** in step "b" centrifugation is, preferably at 84,035 g for 35 minutes at a temperature from 18 to 25 °C; and in step "c", the sugar is selected from the group consisting of trehalose, mannitol, sucrose or lactose, wherein preferably the sugar is used in a ratio of 20:1 (sugar:lipid), and more preferably the sugar is trehalose; and after step "d" of SD, the yield of the drying process, for the formulation obtained, was between 60 and 70%, resulting in particles with a final moisture content between 4 and 5%.

7. Liposomal particles (LP) obtained from the process as defined in any one of claims 1 to 6 **characterized in that** said LP/NCMPs comprises:
- from 50 to 90% in moles of dipalmitoylphosphatidylcholine (DPPC), and from 10 to 50% in moles of 3β-[N-(N',N'-dimethylaminoethane)-carbamoyl]cholesterol (DC-Col), and from 0.00100 to 0 .00775% in moles of an adjuvant molecule consisting of αGalCer forming the organic phase;
- from 0.3 to 2.0 mg of one or more PspA; and
- sugar.

8. Liposomal particles, according to claim 7, **characterized in that** preferably comprises:
- 69.99900% moles of DPPC, 30.00000% moles of DC-Chol, and 0.00100% moles of αGalCer forming the organic phase; and
- 0.3 mg/mL of PspA1; or 0.3 mg/ml of PspA4Pro; or 0.3 mg/mL of 1:1 (PspA1:PspA4Pro).

9. Liposomal particles, according to claim 7 or 8, **characterized in that** PspA is selected from the group consisting of PspA1 (GenBank AY082387), PspA4Pro (GenBank EF649969.1), or a mixture thereof, wherein preferably, PspA1 consists of the amino acid sequence as set out in SEQ ID NO:1, which is translated by the PspA1 gene sequence as set out in SEQ ID NO:2; and PspA4Pro consists of the amino acid sequence as set forth in SEQ ID NO:3, which is translated by the PspA4Pro gene sequence as set out in SEQ ID NO:4.

10. Liposomal particles, according to any one of claims 7-9, **characterized in that** comprise a sugar in the ratio of 20:1 (sugar:lipid), wherein the sugar is selected from the group consisting of trehalose, mannitol, sucrose or lactose, preferably trehalose.

11. Liposomal particles, according to any one of claims 7-10, **characterized in that** the LP/NCMPs:
- are in powder format, dried by atomization spray-drying (SD);
- have average size ranges from 172 to 366 nm, preferably 172 nm;
- have PdI ranges from 0.155 to 0.308, preferably 0.200;
- have zeta potential ranges from +16.93 to +39.95, preferably +38.00; and
- have encapsulation efficiency ranges from 75.04 to 99.99, preferably 90.00%.

12. Liposomal particles, according to any one of claims 7-11, **characterized in that** are for use as a vaccine with comprehensive protection against pneumococcus expressing PspA from family 1 and family 2.

13. Use of liposomal particles formulated as nanocomposite microparticle carriers (LP/NCMPs) as defined in any one of claims 7-12, **characterized in that** it is for the preparation of an immunogenic composition for comprehensive protection against pneumococcus expressing PspA from family 1 and family 2 , wherein the immunogenic composition is preferably a vaccine powder for mucosal administration.

14. Immunogenic composition **characterized in that** it comprises the liposomal particles (LP/NCMPs) as defined in any one of claims 7-12, and a pharmaceutically acceptable carrier and/or adjuvant, wherein the immunogenic composition is preferably a vaccine powder, more preferably to be administered via mucosal route, more preferably targeting the lungs.

15. Immunogenic composition, according to claim 14, **characterized in that** it is for use as a vaccine with comprehensive protection against pneumococcus expressing PspA from family 1 and family 2.
